# EUROPEAN PATENT APPLICATION

(11) **EP 4 818 674 A1**
(43) Date of publication of application: **07.10.2026**
(21) Application number: 25175553.4
(22) Date of filing: 09.05.2025
(51) Int. Cl.: G01N 21/552, G01N 21/77, G01N 21/78, G01N 33/00

(54) **FIBRE OPTIC SENSOR FOR HYDROGEN PLASMONIC SENSING AND INTERROGATION SYSTEM COMPRISING THE SAME**

(30) Priority: 04.04.2025 PT 2025120187
(71) Applicant: INESC TEC - Instituto de Engenharia de Sistemas e Computadores, Tecnologia e Ciência, 4200-465 Porto (PT)
(72) Inventor: Silva Almeida, Miguel Ângelo, 4200-465 Porto (PT); Silva Santos, André David, 4200-465 Porto (PT); Marques Martins de Almeida, José Manuel, 4099-002 Porto (PT); Costa Coelho, Luís Carlos, 4200-465 Porto (PT); Sampaio Mendes, João Pedro, 4200-465 Porto (PT)
(74) Representative: Patentree

(57) **Abstract**

The present disclosure describes an optical sensor, a fibre optic sensor, and an interrogation system for hydrogen plasmonic sensing based on Tamm Plasmon Resonance (TPR). The fibre optic sensor comprises an optical fibre having a distal end on which a TPR multilayer thin-film stack is deposited. The multilayer stack includes a Distributed Bragg Reflector (DBR) formed by a periodic arrangement of dielectric layers, a spacer layer, and a hydrogen-sensitive layer. The plasmonic structure is configured to reflect light according to a resonance condition, which varies in response to hydrogen gas concentration. The DBR preferably comprises two to five periods of alternating silicon dioxide (SiO₂) and titanium dioxide (TiO₂) layers. The disclosure further describes an interrogation system comprising at least one such fibre optic sensor, at least two laser sources emitting at different wavelengths in the infrared range, and at least one photodetector for detecting the reflected signal, enabling accurate and compact hydrogen detection.

## Description

### TECHNICAL FIELD

The present invention relates to the field of gas detection using optical sensing technologies. It particularly concerns systems for hydrogen detection employing infrared spectral interrogation.

### BACKGROUND

In the last few years, hydrogen (H2) has been considered the future clean and renewable energy that will replace traditional fuels, reducing the environmental footprint. This fuel, aligned with solar, wind, and other renewable energy sources already implemented, has the potential to be applied in several circumstances [1,2]. In fact, 156 countries announced plans involving this green energy to achieve carbon neutrality during the 2021 Glasgow summit [3,4].

The advantages of H2 are the high mass-energy density, the easy production by water splitting using renewable energy, and the fact that it is easily stored and transported over high distances using pipelines. Furthermore, from production to usage, carbon dioxide emissions can be entirely eliminated [2,5]. Despite all the advantages, this fuel has a critical disadvantage: the high flammability and explosive nature with low ignition energy at concentrations from 4 to 75 vol% [6]. In this way, the correct monitoring of possible leaks is essential for safer use on the daily day [2,3,7,8].

In this context, the academic community has explored several types of H₂ sensors, such as electrical, magnetic, and optical sensors, among others [3,9,10]. In particular, optical sensors are the type of sensors with more advantages to be applied along the pipelines with a fast and remote interrogation, without the ignition sources and immune to electromagnetic interference [11]. Despite this and its high sensitivity, the high cost of the produced sensors and the short operational lifespan have limited commercial adoption, motivating the development of new technologies [12]. The different types of optical techniques most used are micro-mirror sensors [13], colorimetric sensors [14], fibre optical sensors [15,16], and plasmonic sensors [17-19]. The latter can be divided into two main techniques, Surface Plasmon Resonance (SPR) and Localized SPR (LSPR), where the difference is the use of thin films and nanoparticles, respectively.

Both techniques are based on the transfer of energy from light to collective oscillations of electrons, called plasmons, between a medium with negative permittivity (metal) and a medium with positive permittivity (dielectric). The light energy will be absorbed at the resonant conditions, resulting in a dip in the reflected light [7,20]. These conditions depend on the dielectric constants of the metal and dielectric material and the dielectric constant of the external medium. In this way, for H₂ sensing, palladium (Pd) or other metal hydrides are usually used as an external medium. In particular, Pd has a high ability to absorb H₂, changing the optical and mechanical properties with high reversibility [21-23]. Therefore, this metal becomes a dielectric material, PdHₓ, in the presence of the gas, changing the resonant wavelength of the plasmonic structure and allowing the H₂ detection.

The current works presented in the literature based on SPR are composed of a structure of three layers. The first one is composed of a plasmonic material (Au or Ag), then a dielectric layer (TiO₂, SiO₂, or Ta₂O₅), and the last one with an H₂-sensitive material (Pd or Pt) [17,24]. The latter must be of very low thickness (around 3nm) due to the high losses of the metal hydrides. With this type of structure, it is possible to achieve wavelength shifts up to 28nm [17], but with very wide spectral attenuation bands and with the optimized bands in the visible range.

More recently, several works involving Pd and Pd-alloys nanoparticles, or core-shell nanoparticles, demonstrated the possibility of exciting LSPR bands in the infrared range. For example, Yip *et al.* [25] with AuPd nanobipyramids and Rodal-Cedeira *et al.* [19] with AuPd nanorods. obtained an LSPR wavelength shift of 140nm at 2 vol% H₂ and 40nm at 1 vol% H₂, respectively, with the LSPR band around 1000 and 1200nm. Despite the good sensitivity, these traditional and well-known plasmonic sensors have several disadvantages, such as complex production methods, expensive materials (Au and Ag), and the need for p-polarized light.

Following this idea, in 2023, two promising works were published based on Tamm Plasmon Resonance (TPR) with some preliminary experimental results [26,27]. The TPR structures are composed of a Distributed Bragg Reflector (DBR) with a metal film, which for H₂ detection is Pd or other metal hydrides. The DBR can be composed of a periodic structure with two dielectric materials with different refractive indices. The thickness of the layers defines the spectral position of the DBR stopband, following the equation *RI·d* = *λ*/*4,* where *RI* is the refractive index of the material, d is the thickness, and *λ* the centre wavelength of the stopband [28]. This plasmonic mode can be excited with normal incidence and with depolarized light, which is an advantage over traditional plasmonic methods. In addition, the basis of the structure is composed of cost-effective materials that can be easily deposited instead of expensive plasmonic materials.

These facts are disclosed in order to illustrate the technical problem addressed by the present disclosure.

### References

[1] A.I. Osman, N. Mehta, A.M. Elgarahy, M. Hefny, A. Al-Hinai, A.H. Al-Muhtaseb, D.W. Rooney, Hydrogen production, storage, utilisation and environmental impacts: a review, Environ Chem Lett 20 (2022) 153-188. https://doi.org/10.1007/s10311-021-01322-8.
[2] M.H. McCay, S. Shafiee, Hydrogen: An energy carrier, in: Future Energy: Improved, Sustainable and Clean Options for Our Planet, Elsevier, 2020: pp. 475-493. https://doi.org/10.1016/B978-0-08-102886-5.00022-0.
[3] A.K. Pathak, S. Verma, N. Sakda, C. Viphavakit, R. Chitaree, B.M.A. Rahman, Recent Advances in Optical Hydrogen Sensor including Use of Metal and Metal Alloys: A Review, Photonics 10 (2023) 122. https://doi.org/10.3390/photonics10020122.
[4] J.S. Lee, J.W. An, S. Bae, S.K. Lee, Review of Hydrogen Gas Sensors for Future Hydrogen Mobility Infrastructure, Applied Science and Convergence Technology 31 (2022) 79-84. https://doi.org/10.5757/ASCT.2022.31.4.79.
[5] R. Singh, M. Singh, S. Gautam, Hydrogen economy, energy, and liquid organic carriers for its mobility, in: Mater Today Proc, Elsevier Ltd, 2020: pp. 5420-5427. https://doi.org/10.1016/j.matpr.2020.09.065.
[6] Y. Lee, M.H. Cho, M.C. Lee, Y.J. Kim, Evaluating hydrogen risk management policy PR: Lessons learned from three hydrogen accidents in South Korea, Int J Hydrogen Energy 48 (2023) 24536-24547. https://doi.org/10.1016/j.ijhydene.2023.03.142.
[7] B. Ai, Y. Sun, Y. Zhao, Plasmonic Hydrogen Sensors, Small 18 (2022). https://doi.org/10.1002/smll.202107882.
[8] L. Bannenberg, H. Schreuders, B. Dam, Tantalum-Palladium: Hysteresis-Free Optical Hydrogen Sensor Over 7 Orders of Magnitude in Pressure with Sub-Second Response, Adv Funct Mater (2021). https://doi.org/10.1002/adfm.202010483.
[9] I.S. Maksymov, M. Kostylev, Magneto-Electronic Hydrogen Gas Sensors: A Critical Review, Chemosensors 10 (2022). https://doi.org/10.3390/chemosensors10020049.
[10] I. Darmadi, F.A.A. Nugroho, C. Langhammer, High-Performance Nanostructured Palladium-Based Hydrogen Sensors - Current Limitations and Strategies for Their Mitigation, ACS Sens 5 (2020) 3306-3327. https://doi.org/10.1021/acssensors.0c02019.
[11] Y. nan Zhang, H. Peng, X. Qian, Y. Zhang, G. An, Y. Zhao, Recent advancements in optical fiber hydrogen sensors, Sens Actuators B Chem 244 (2017) 393-416. https://doi.org/10.1016/j.snb.2017.01.004.
[12] G. Manjavacas, B. Nieto, Hydrogen sensors and detectors, in: Compendium of Hydrogen Energy, Elsevier, 2016: pp. 215-234. https://doi.org/10.1016/b978-1-78242-364-5.00010-5.
[13] J. Karst, M. Hentschel, F. Sterl, H. Linnenbank, M. Ubl, H. Giessen, Optimizing magnesium thin films for optical switching applications: rules and recipes, Opt Mater Express 10 (2020) 1346. https://doi.org/10.1364/ome.393832.
[14] S. Hwan Cho, J. Min Suh, B. Jeong, T. Hyung Lee, K. Soon Choi, T. Hoon Eom, T. Kim, H. Won Jang, Fast responding and highly reversible gasochromic H2 sensor using Pd-decorated amorphous WO3 thin films, Chemical Engineering Journal 446 (2022). https://doi.org/10.1016/j.cej.2022.136862.
[15] L. Coelho, J.M.M.M. de Almeida, J.L. Santos, D. Viegas, Fiber optic hydrogen sensor based on an etched Bragg grating coated with palladium, Appl Opt 54 (2015) 10342. https://doi.org/10.1364/ao.54.010342.
[16] C. Zhang, C. Shen, X. Liu, S. Liu, H. Chen, Z. Huang, Z. Wang, T. Lang, C. Zhao, Y. Zhang, Pd/Au nanofilms based tilted fiber Bragg grating hydrogen sensor, Opt Commun 502 (2022). https://doi.org/10.1016/j.optcom.2021.127424.
[17] A. Hosoki, M. Nishiyama, H. Igawa, A. Seki, Y. Choi, K. Watanabe, A surface plasmon resonance hydrogen sensor using Au/Ta2O 5/Pd multi-layers on hetero-core optical fiber structures, Sens Actuators B Chem 185 (2013) 53-58. https://doi.org/10.1016/j.snb.2013.04.072.
[18] C. Wadell, F.A.A. Nugroho, E. Lidström, B. landolo, J.B. Wagner, C. Langhammer, Hysteresis-free nanoplasmonic pd-au alloy hydrogen sensors, Nano Lett 15 (2015) 3563-3570. https://doi.org/10.1021/acs.nanolett.5b01053.
[19] S. Rodal-Cedeira, V. Montes-García, L. Polavarapu, D.M. Solís, H. Heidari, A. La Porta, M. Angiola, A. Martucci, J.M. Taboada, F. Obelleiro, S. Bals, J. Pérez-Juste, I. Pastoriza-Santos, Plasmonic Au@Pd Nanorods with Boosted Refractive Index Susceptibility and SERS Efficiency: A Multifunctional Platform for Hydrogen Sensing and Monitoring of Catalytic Reactions, Chemistry of Materials 28 (2016) 9169-9180. https://doi.org/10.1021/acs.chemmater.6b04941.
[20] V. Yesudasu, H.S. Pradhan, R.J. Pandya, Recent progress in surface plasmon resonance-based sensors: A comprehensive review, Heliyon 7 (2021). https://doi.org/10.1016/j.heliyon.2021.e06321.
[21] K.J. Palm, J.B. Murray, T.C. Narayan, J.N. Munday, Dynamic Optical Properties of Metal Hydrides, ACS Photonics 5 (2018) 4677-4686. https://doi.org/10.1021/acsphotonics.8b01243.
[22] C. Wadell, S. Syrenova, C. Langhammer, Plasmonic hydrogen sensing with nanostructured metal hydrides, ACS Nano 8 (2014) 11925-11940. https://doi.org/10.1021/nn505804f.
[23] K. Chen, D. Yuan, Y. Zhao, Review of optical hydrogen sensors based on metal hydrides: Recent developments and challenges, Opt Laser Technol 137 (2021). https://doi.org/10.1016/j.optlastec.2020.106808.
[24] C. Perrotton, R.J. Westerwaal, N. Javahiraly, M. Slaman, H. Schreuders, B. Dam, P. Meyrueis, A reliable, sensitive and fast optical fiber hydrogen sensor based on surface plasmon resonance, 2013.
[25] H.K. Yip, X. Zhu, X. Zhuo, R. Jiang, Z. Yang, J. Wang, Gold Nanobipyramid-Enhanced Hydrogen Sensing with Plasmon Red Shifts Reaching =140 nm at 2 vol% Hydrogen Concentration, Adv Opt Mater 5 (2017). https://doi.org/10.1002/adom.201700740.
[26] Y. Chen, Z. Yang, M. Ye, W. Wu, L. Chen, H. Shen, S. Ishii, T. Nagao, K. Chen, Tamm Plasmon Polaritons Hydrogen Sensors, Advanced Physics Research 2 (2023). https://doi.org/10.1002/apxr.202200094.
[27] K. Zhang, Z. Chen, H. Li, Z. Yi, Y. Liu, X. Wu, A dual-band hydrogen sensor based on Tamm plasmon polaritons, Physical Chemistry Chemical Physics 25 (2023) 20697-20705. https://doi.org/10.1039/D3CP02653A.
[28] C. Kar, S. Jena, D. V. Udupa, K.D. Rao, Tamm plasmon polariton in planar structures: A brief overview and applications, Opt Laser Technol 159 (2023). https://doi.org/10.1016/j.optlastec.2022.108928.

### GENERAL DESCRIPTION

The present invention relates to a fibre-optic sensor device and interrogation system designed for hydrogen detection across a wide range of applications. The sensor is particularly suited for integration into hydrogen pipelines, enabling remote interrogation and real-time detection of hydrogen leaks or accumulations along extended infrastructure. It is also applicable to hydrogen storage facilities, where continuous and distributed monitoring is critical for safety and operational efficiency. Furthermore, the fibre sensor enables hydrogen detection and monitoring at spatially separated refuelling posts or stations, supporting decentralized energy infrastructure.

To facilitate the application of remote sensing and allow the use of the most cost-effective equipment based on infrared technology, the present disclosure describes a structure with a TPR band, in particular between 1500 and 1600nm. In a first aspect, a comprehensive numerical analysis is conducted to evaluate the influence of several parameters on the TPR band (number of periods, spacer and metallic thickness, and angle, among others). Subsequently, based on the optimized configuration, a sensor structure is developed and characterized under varying hydrogen concentrations, determining the response time, sensitivity, stability, and resistance to interfering substances.

The present disclosure relates to an optical fibre sensor, in particular a Tamm Plasmon Resonance (TPR)-based sensor structure specifically engineered to operate in the infrared range, particularly between 1500 and 1600 nm, using monomode optical fibres. Operating in this spectral region offers several practical advantages, including compatibility with low-cost infrared components and enhanced suitability for remote sensing applications. However, designing a TPR structure in the infrared presents unique challenges due to increased material dispersion, optical losses, and the need for precise control of field confinement.

To overcome these limitations, a complete numerical analysis was conducted to investigate the influence of key structural parameters, such as the number of periods in the Distributed Bragg Reflector (DBR), spacer layer thickness, metal layer thickness, and angle of incidence. Based on the optimized design, a functional sensor was fabricated and experimentally validated for hydrogen detection. The resulting device demonstrates high sensitivity, rapid response time, strong resistance to interferents, and long-term stability, confirming its potential for practical and scalable hydrogen sensing applications.

The present disclosure relates to a hydrogen sensor based on optical fibre technology, suitable for deployment in large-scale industrial infrastructures, such as pipelines or storage facilities. In an embodiment, the sensor system comprises one or more optical fibres functionalized at their distal ends with a plasmonic nanostructure that serves as a sensing probe. The nanostructure operates based on the Tamm Plasmon Resonance (TPR) phenomenon and includes a thin, gas-sensitive film whose optical properties undergo a measurable change upon exposure to hydrogen gas. This change results in a spectral shift in the plasmonic resonance band, which may be detected by analysing variations in the intensity of light reflected from the sensor tip.

In an embodiment, the plasmonic structure comprises a periodic multilayer arrangement of dielectric materials, such as silicon dioxide (SiO₂), titanium dioxide (TiO₂), or other materials having different refractive indices. The gas-sensitive film may be formed from a metal hydride, including but not limited to pure palladium (Pd), magnesium (Mg), or alloys thereof. To enhance durability and environmental stability, the sensitive film can be coated with a polymeric protection layer, which is selected so as not to interfere with the optical performance of the sensor.

The detection method, in an embodiment, involves monitoring the intensity variation of light reflected from the fibre tip when illuminated by two or more laser sources. These laser wavelengths are selected to fall on opposite slopes of the plasmonic resonance band. This configuration enables the system to distinguish between signal variations caused by hydrogen and those arising from unrelated external stimuli, thereby reducing the risk of false positives. Specifically, hydrogen interaction results in a differential intensity change - an increase at one wavelength and a decrease at the other - whereas common noise sources tend to cause similar directional changes in both signals.

In one embodiment, control of the laser sources, acquisition of the reflected light signals, and processing of the sensor data is performed by a dedicated software application. This software may provide a user-friendly graphical interface for sensor control, real-time monitoring, and data visualization, facilitating ease of use in field or industrial settings.

The system, in some embodiments, utilizes single-mode optical fibres operating in standard telecommunications bands, such as the S-, C-, or L-bands. This allows for high-speed, remote sensing over long distances-potentially tens of kilometres-while taking advantage of low-cost and widely available telecommunications components, including laser sources and photodetectors. The engineering of the plasmonic structure enables resonance within the infrared region, specifically between 1500 nm and 1600 nm, which differs from conventional plasmonic sensors typically operating in the visible spectrum.

Furthermore, in an embodiment, the system architecture supports multiplexed sensing, enabling multiple fibre sensors to be interrogated sequentially through automated switching. This feature allows for scalable deployment across large sensing networks, offering both high spatial resolution and reduced operational cost.

In addition, the use of primarily dielectric materials in the plasmonic structure, as opposed to noble metals such as gold or silver, provides a cost advantage. In certain embodiments, this results in a sensor that is not only highly sensitive and scalable but also more economically viable for widespread industrial use.

An aspect of the present disclosure relates to a fibre optic sensor for hydrogen plasmonic sensing, comprising an optical fibre having a distal end and a Tamm Plasmon Resonance, TPR, multilayer thin-film stack arranged on the distal end of the optical fibre, wherein the TPR multilayer thin-film stack comprises:
a periodic arrangement of dielectric material layers forming a Distributed Bragg Reflector, DBR;
a hydrogen-sensitive layer; and
a dielectric spacer layer between the DBR and the hydrogen-sensitive layer;
wherein the plasmonic structure is configured to reflect light according to a resonance condition, and
wherein the hydrogen-sensitive layer is configured to induce a change in said resonance condition according to the concentration of hydrogen gas.

In an embodiment, the Distributed Bragg Reflector comprises two to five periods of dielectric material layers, preferably three periods of dielectric material layers.

In an embodiment, each period of dielectric material layers comprises a silicon dioxide, SiO₂, layer and a titanium dioxide, TiO₂, layer.

In an embodiment, the spacer is a silicon dioxide, SiO₂, layer.

In an embodiment, the hydrogen-sensitive layer comprises a metal, or metal alloy, forming a metal hydride in the presence of hydrogen.

In an embodiment, the metal, or metal alloy, is selected from selective materials such as palladium (Pd), magnesium (Mg), or alloys thereof.

In an embodiment, the fibre further comprises a protective layer covering the hydrogen-sensitive layer, in particular said protective layer being formed of a polymeric material that is permeable to hydrogen gas.

In an embodiment, the optical fibre is a single-mode optical fibre.

In an embodiment, the measurable change in resonance condition is determined by monitoring variations in the intensity of reflected light at one or more interrogation wavelengths, in particular two interrogation wavelengths.

In an embodiment, the resonance condition is a resonance band, in particular a frequency-dependent absorption band.

In an embodiment, the interrogation wavelengths comprise at least two interrogation wavelengths positioned on opposite slopes of the resonance band.

In an embodiment, the plasmonic resonance band is located in the infrared spectral region between 1200 nm and 1700 nm.

A further aspect of the present disclosure relates to an interrogation system for hydrogen plasmonic sensing, comprising:
at least one fibre optic sensor;
at least two laser sources, each configured to emit light at a different wavelength, preferably within the range of 1300 nm of 1600 nm; preferably 1500 nm to 1600 nm;
at least one photodetector configured to receive light reflected from the fibre optic sensor and to generate a corresponding electrical signal.

In an embodiment, the interrogation system further comprises optical switching modules configured to allow selection of one or more of the lasers; and configured to select between a plurality of fibre optic sensors to interrogate.

In an embodiment, the interrogation system further comprises a microcontroller configured to control system operation and communicate with an external computing device.

In an embodiment, the interrogation system further comprises a laser driver circuit configured to supply controlled and stable electrical currents to laser diodes.

In an embodiment, the interrogation system further comprises a temperature control circuit configured to regulate the temperature in the vicinity of the laser diodes to stabilize their emission wavelengths.

In an embodiment, the microcontroller is further configured to manage laser channel selection, optical switching, signal acquisition, and data transmission to the computing device.

A further aspect of the present disclosure relates to an optical sensor for hydrogen plasmonic sensing using infrared wavelengths, comprising a Tamm Plasmon Resonance, TPR, multilayer thin-film stack.
wherein the TPR multilayer thin-film stack comprises:
a periodic arrangement of dielectric material layers forming a Distributed Bragg Reflector, DBR;
a hydrogen-sensitive layer; and
a dielectric spacer layer between the DBR and the hydrogen-sensitive layer;
wherein the plasmonic structure is configured to reflect light according to a resonance condition, and
wherein the hydrogen-sensitive layer is configured to induce a change in said resonance condition according to the concentration of hydrogen gas;
wherein the DBR comprises two to five periods of dielectric material layers, preferably three periods of dielectric material layers;
wherein each period of dielectric material layers comprises a silicon dioxide, SiO₂, layer and a titanium dioxide, TiO₂, layer.

In an embodiment, the spacer is a silicon dioxide, SiO₂, layer.

In an embodiment, the hydrogen-sensitive layer comprises a metal, or metal alloy, forming a metal hydride in the presence of hydrogen.

In an embodiment, the metal, or metal alloy, is selected from palladium (Pd), magnesium (Mg), or alloys thereof.

In an embodiment, the optical sensor further comprises a protective layer covering the hydrogen-sensitive layer, in particular said protective layer being formed of a polymeric material that is permeable to hydrogen gas.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of a fibre optic sensor comprising a plasmonic nanostructure positioned on the distal end of a single-mode optical fibre, according to an embodiment of the present disclosure.
**Figure 2****:** Illustrates a diagram depicting the behaviour of reflected laser signal intensities under different sensing conditions. In the illustrated example, the incident laser wavelengths are 1505 nm and 1587 nm, respectively.
**Figure 3****:** Illustrates a block diagram of a possible configuration for a system for controlling at least one fibre optic sensor according to the present disclosure. Lines with arrowheads represent electrical connections, while lines without arrowheads represent optical connections.
**Figure 4****:** Illustrates the spectrum of a DBR stopband and three different TPR modes. The difference between the TPR bands relies on the spacer and metallic layer's thickness. On the right side, a schematic TPR structure is composed of a three-period DBR, a spacer, and an H2-sensitive metal layer (Pd). The light is applied through the substrate of the structure.
**Figure 5****:** Graphs depicting the influence of spacer and metal thickness on the TPR band position: a) spacer influence for three periods, b) metal influence for three periods, c) spacer influence for four periods, d) metal influence for four periods. The blue areas corresponding to the minimums of the reflectivity, namely to the minimum of the TPR band.
**Figure 6****:** Graphs depicting the electric field, |E/E₀|, in the TPR structure. The confinement of the electric field occurs between the last DBR layer and the spacer; a) Wavelength dependence of the |E/E₀|; b) |E/E₀| for the resonance wavelength (1572nm) and a wavelength outside the TPR band (1400nm).
**Figure 7****:** Graphs depicting the influence of the incidence angle for both light polarizations: a) s-polarized light; b) p-polarized light.
**Figure 8****:** Graphs depicting the H₂ sensitivity of the TPR structure: a) impact of the presence of H₂ on the TPR band position (solid lines correspond to the absence of H₂, and dashed lines to the presence of H₂ on the Pd structure); b) Sensitivity (wavelength shift of the TPR band) and c) FOM for different spacer and metal thicknesses.
**Figure 9****:** Schematic illustrates the experimental setup for sensor characterization, including flow controllers, a light source, and a spectrometer connected to a sealed external chamber. An internal chamber within it allows direct gas application to the sensor surface. Upper and frontal views of the internal chamber are shown, along with a real image of the sensor positioned perpendicular to the incident light.
**Figure 10**: a) Images of the produced structures: i) optical image of the substrate with a DBR deposit on the surface; ii) SEM image of the complete structure with three DBR's periods, spacer, Pd, and PTFE; b) Reflection spectra of the deposited structures in comparison with the simulated DBR with the same thicknesses; c) TPR bands of the Tamm 1, 2, and 3 without (solid line) and with H₂ (dashed line).
**Figure 11****:** Graphs depicting a) complete cycles with a sequence of atmospheric air - N₂ - H₂ - N₂ using 1 and 4 vol% of H₂. The sensitivity obtained was 0.9 and 9.5nm for 1 and 4 vol% of H₂, respectively; b) calculated sensor response times (transition between N₂ and H₂); c) calculated sensor reset times (transition between H₂ and N₂).
**Figure 12****:** Graphs depicting sensor's response for different H₂ concentrations, where the nonlinearity of Pd response is presented; b) Analysis of the Pd phases for an easy interpretation of the non-linear behaviour; and c) sensor response to consecutive cycles with N₂ and 4 vol% H₂.
**Figure 13****:** Illustrates the sensor's interaction with possible interferents: a) 2500ppm CH₄, and b) 500ppm NH₃. No cross-sensitivity being observed.
**Figure 14****:** Graphs illustrating the spacer thickness influence on the TPR band for different numbers of periods using a metal layer with 30nm: a) two periods, b) three periods, c) four periods, and d) five periods.
**Figure 15****:** Graphs illustrating metal thickness influence on the TPR band for different numbers of periods using the spacer layer with 250nm: a) two periods, b) three periods, c) four periods, and d) five periods.
**Figure 16****:** Graphs of a) FWHM of the TPR band for different spacer and metallic layer thicknesses; b) Study of the influence of protection polymers and adhesion layer on the TPR band.
**Figure 17****:** Graphs of a) minimum of the TPR band without and with 4 vol% H₂, with a wavelength shift of 9 nm; b) reflectivity variation between the TPR band without and with H₂.
**Figure 18****:** Graphs illustrating the sensor's response for different H₂ concentrations through diluting of a) 1 and b) 4 vol% H₂. c) Reflectivity variation of the resonance wavelength for 10 consecutive cycles.
**Figure 19****:** Graphs depicting TPR bands in different ambient temperatures between 23 and 65°C.

### DETAILED DESCRIPTION

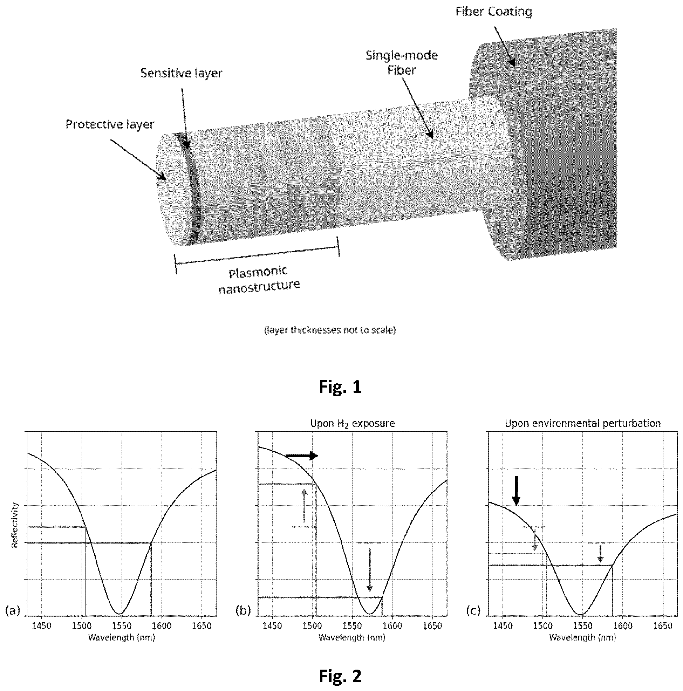

According to the present disclosure, the fibre sensor as represented in **Figure 1****,** comprises a plasmonic structure including a DBR, a spacer, and a sensitive layer (i.e. a metal layer), and a protective layer. Measurement of variations in the reflectance spectrum of the plasmonic nanostructure is performed with two continuous-wave laser signals at different wavelengths. These lasers should be positioned on either side of the Tamm Plasmon Resonance which, in a preferred embodiment, lies between 1500 and 1600 nm. However, the system can be implemented across a broader range - from the visible to the near infrared due to the tuneable capabilities of the system.

**Figure 2** schematically shows the behaviour of the reflected optical intensities at the laser wavelengths, which are conditioned by the nanostructure's spectrum, shown with a solid black line. The other lines indicate the lasers' wavelengths and positions on the spectrum as well as the amount of optical power reflected relative to incident power, on the y-axis.

When the nanostructure is exposed to hydrogen (Figure 2 (b)), the change in its optical properties causes the band to "shift" into greater wavelengths - this means that the reflected signal from the lower-wavelength laser intensifies, while the other decreases in magnitude. Likewise, the return to an absence of hydrogen would bring about the reverse process, reestablishing the levels in Figure 2 (a).

On the other hand, undesired stimuli affecting the sensor (possible damage or degradation to the fibre and/or the nanostructure, placement of the sensor and fibre curvature, environmental conditions, etc.) cause the reflectivity to almost uniformly increase or decrease. In this case, both reflected signals increase or decrease, respectively - Figure 2 (c).

Therefore, the signals can be easily monitored, allowing one to determine, by continuously comparing their change, if reflectivity variations are due to the presence of hydrogen or due to some other stimulus that may not be of interest.

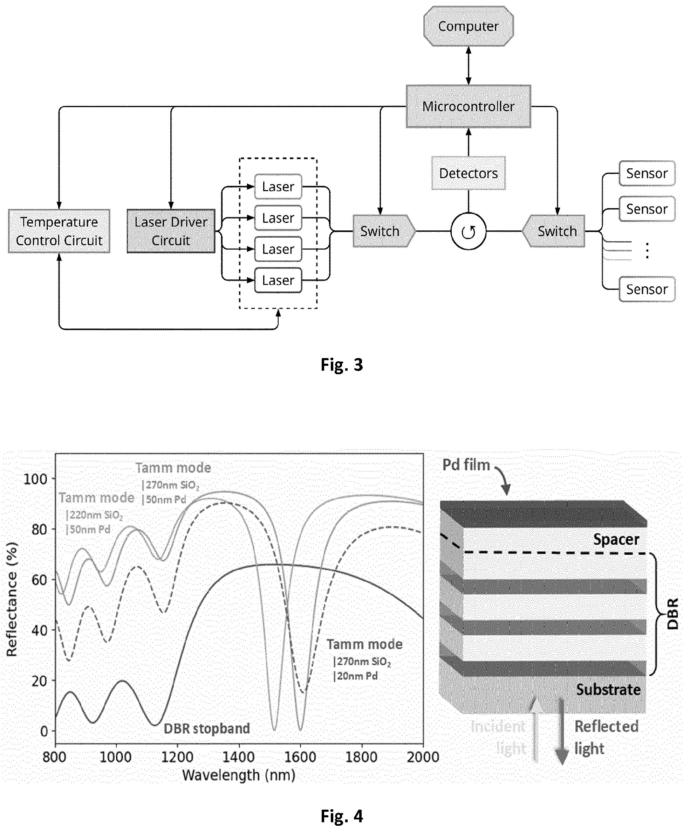

**Figure 3** highlights a block diagram of an interrogation system for control and measurement, comprising several key components designed to ensure precise operation and data acquisition. In an embodiment the system includes a microcontroller board that interfaces with a computer and manages various subcircuits and switches. The system includes a laser driver circuit capable of delivering stable and controlled electrical currents to multiple laser diodes. A temperature control circuit is integrated to maintain thermal stability around the lasers, thereby preserving their central emission wavelengths. In an embodiment, optical switches are employed to enable the selection of individual laser channels and the specific sensor (fibre tip) to be interrogated. The setup features multiple laser diodes operating in the near infrared wavelength range. While the 1500-1600 nm range offers optimal performance for remote sensing applications, the system is designed to function effectively across a broader spectral range. Detectors and lasers used are fully compatible with this spectral region.

**Figure 4** shows different reflectance spectra for different structures and an example of a possible TPR structure, in accordance with an embodiment of the present disclosure. This structure is composed of a DBR with three periods of TiO2/SiO2, a spacer of SiO2, and a metallic film of Pd. The incidence of light, in this case, occurs through the substrate.

In an embodiment, the thickness of the DBR's layers is equal to 168.5 nm and 265.4 nm for the TiO2 (RI = 2.3) and SiO2 (RI=1.46), respectively. These thicknesses generate a stopband around 1550nm, as observed in the graph of Figure 4. With three periods, the maximum reflectivity is approximately 60%, however, this value can increase by adding more periods. In the same Figure, it is possible to observe three different Tamm modes, where the difference relies on the thickness of the spacer and metal layer. These results show that the higher thickness of the spacer induces a redshift, and a thin layer of Pd increases the minimum of reflectivity. With 50nm of Pd, the difference between the maximum and minimum values is around 90%, which is a substantial improvement over the traditional SPR structures.

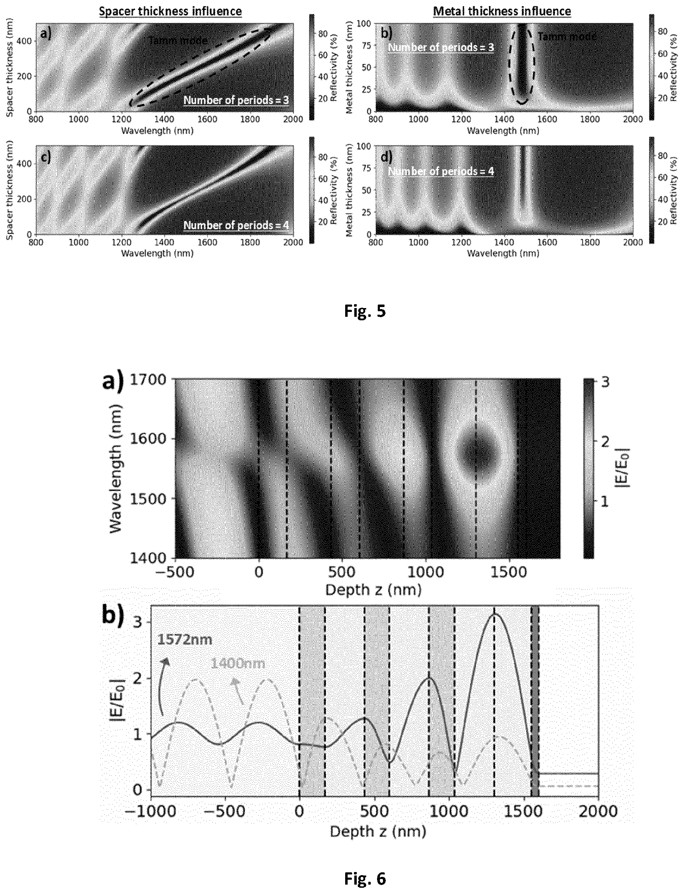

To better understand the influence of the spacer and metallic layer and the number of periods on the definition and position of the TPR band, different colormaps were designed to represent the reflectivity in the function of these parameters. Figure 5-a) shows the reflectivity for three periods, 50 nm of Pd, and different spacer thicknesses. The spectra range between 1200 and 2000nm corresponds to the DBR stopband range, and inside, it is possible to observe a blue zone. This zone corresponds to a minimum, namely the Tamm mode, which means that the increases in the spacer thickness induce a redshift on the TPR band with a linear relation. This graph is important because it demonstrates the possibility of tuning this plasmonic mode for the required wavelength inside the stopband.

Figure 5-b) follows the same concept but as a function of the metal thickness while maintaining the spacer thickness equal to 200nm. In this case, the graphs show us independence between the spectral position of the TPR band and the metal thickness but a strong influence on the definition of the band, mainly for low thicknesses. On TPR, the electric-field confinement occurs due to the existence of a negative dielectric constant. When the thickness of metal is too thin, the effective dielectric constant of metal/air will not be enough to confine the electrical field. This limit occurs for thicknesses lower than 20nm, where it is possible to observe that any plasmonic mode is excited.

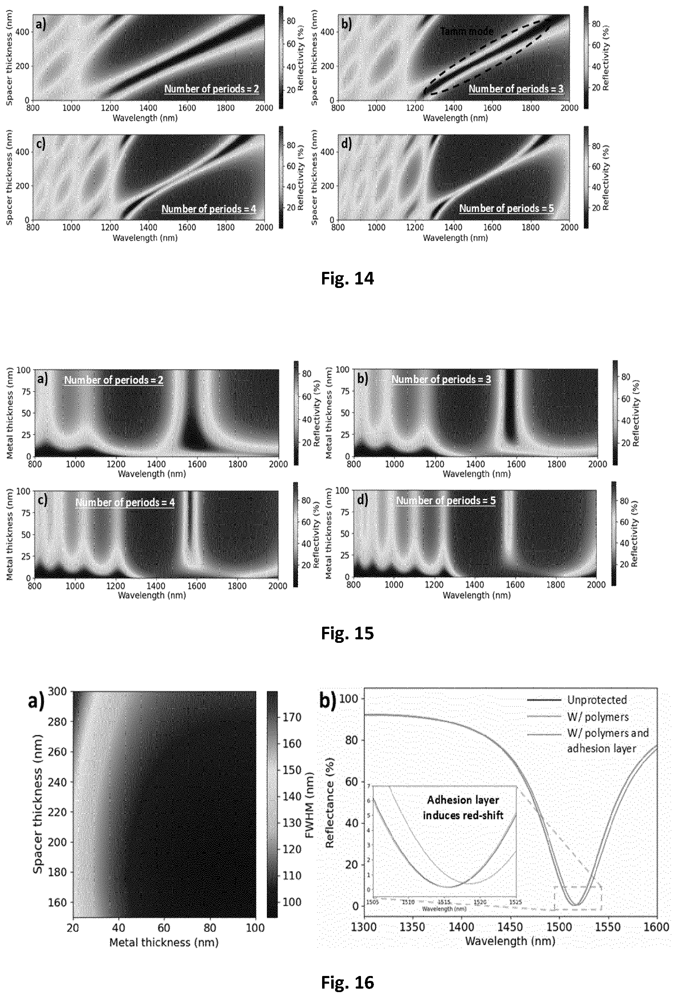

In Figures 5-c) and d) are represented similar results but with four periods of TiO₂/SiO₂. Colour maps for structures with different periods (between two and five periods) are also presented - see Figures 14 and 15. Increasing the number of periods increases the DBR definition and reflectivity, resulting in TPR bands with narrower attenuation bands. However, the high reflectivity of the DBR increases the minimum of the band, decreasing the difference between the minimum and maximum reflectivity. For five periods, in Figure 14-d) and 15-d), the TPR mode almost disappears with a minimum reflectivity of around 60%. With the decrease of periods, in Figure 14-a) and 15-a), very large attenuation bands (around 200 nm) are observed. In this way three DBR periods are the preferred value of periods.

In Figure 6 the magnitude of the electric field is depicted, |E/E₀|, in depth in the structure. In Figure 6-a) this value is represented in function of the wavelength, observing confinement of the electric field around 1572 nm that corresponds to the minimum of the TPR band. Figure 6-b) shows another representation of the electric field but for the resonance wavelength (1572 nm) and a wavelength outside of the TPR band (1400 nm). For 1572 nm, we observe a maximum value on the interface between the DBR and the spacer, which decays fast until it penetrates the metal layer. For the 1400 nm, we observe the normal behaviour of a DBR, which corresponds to an increase of the electric field on the substrate and a quasi-zero value after the metal layer due to the high reflectivity.

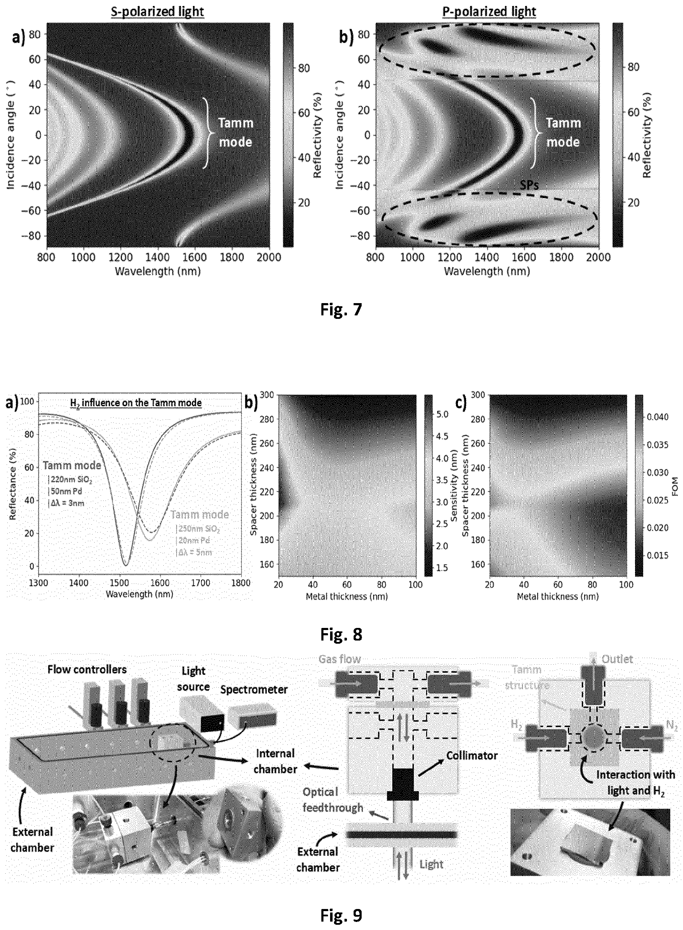

The incidence angle also influences the TPR plasmonic mode. This influence is represented in **Figure 7****.** It is important to separate the two polarizations of light, considering that different polarizations excite different behaviours at high angles. As seen in Figure 7, for normal incidence, the two polarizations have the same spectra, which proves the ability to use depolarized light to excite TPR. However, the increase in the incidence angle induces a blueshift in the TPR band, and the s- and p-polarization starts to distinguish. For the s-polarized light, in Figure 7-a), the Tamm mode blueshift until the complete disappearance, without any plasmonic modes at high angles. For the p-polarized light in Figure 7-b) it is also possible to observe a blueshift, but new plasmonic modes start to appear. Considering that these modes do not appear with the s-polarized light and that occurs for angles higher than the critical angle (42°), it is possible to conclude that these modes correspond to Surface Plasmons (SPs). In this way, the normal incidence is preferential to exciting TPR modes. However, small incidence angles (that can occur in experimental setups due to experimental errors) will have a negligible impact on the band position.

The following numerical analysis assesses how the structure responds to the presence of H₂. For this, the TPR bands considering the dielectric properties of Pd and PdH_{0.65} were calculated, and the sensitivity of the structure is the wavelength shift between these two bands. In Figure 8-a) are represented two pairs of TPR bands with different morphologies. In both pairs, a redshift of the band and an increase in the minimum reflectivity is observed. This behaviour is related to the transformation of Pd from metal to dielectric. As previously indicated, a negative dielectric constant is needed to induce the electric field confinement. When the PdHₓ becomes dielectric, the confinement of the light decreases, increasing the reflected light at the minimum point.

Comparing the TPR band at 1510 nm and 1590 nm, the latter has higher sensitivity but presents a higher bandwidth. To optimize the structure for H₂ sensing, the sensitivity, Full-Width-Half-Maximum (FWHM), and Figure-of-Merit (FOM) were calculated from different spacer and metal thicknesses. Figure 8-b) represents the sensitivity. In this graph, a higher sensitivity of approximately 5 nm is observed for low metal thicknesses and with a spacer thickness between 210 and 250 nm, which corresponds to the TPR band centred in the DBR stopband. The higher sensitivity occurs due to the low confinement of the electric field on the spacer layer, which allows a higher penetration of the field in the metal layer.

When the metal layer is high enough, the confinement of the field is high and presents a substantial decay in the space, with low magnitude in the Pd layer, as observed in **Figure 6****-b.** However, due to the low confinement, these thicknesses are also the thicknesses with a wide attenuation band (see Figure 16-a)). Considering that the FOM is the ratio between these two parameters, the preferred structures occur for higher metal thicknesses, as seen in Figure 8-c), with a maximum FOM of 0.04.

To conclude the numerical analysis, the impact of adhesion layers or protection layers was studied. Regarding the adhesion layer, a chromium (Cr) layer with 3 nm was considered. The graph of Figure 16-b) shows us that the introduction of this layer induces a redshift of the TPR band of approximately 3 nm due to the lower metallic nature of Cr compared with Pd. Regarding the protection layers, two materials were considered, namely, 35 nm of polytetrafluoroethylene (PTFE) and 30nm of poly(methylmethacrylate) (PMMA). These polymers are used to protect the Pd film from humidity and carbon monoxide and decrease the time response. As observed in Figure 16-b), adding these layers has a minimal impact on the band position. Hence, their application is viable for protecting the sensing material without interfering with the plasmonic properties.

Following the numerical analysis, the preferred structures were deposited in planar substrates using sputtering deposition. As shown in Figure 9, the substrate was placed inside an internal chamber with a collimator to obtain normal incidence. This internal setup is inside of an external chamber for safety reasons. The light reaches the TPR structure through the substrate, and the gas flow (monitored by flow controllers) is applied on the metal side to correctly interact with Pd. The reflected light is collected by an infrared spectrometer for further analysis.

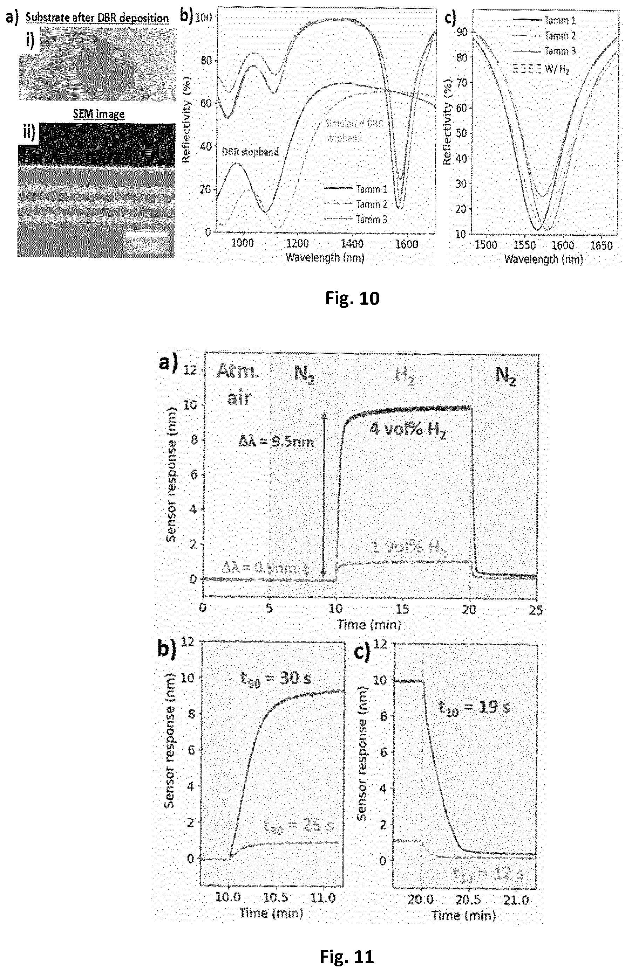

In **Figure 9****,** a real image of the TPR structure from the metal side is also shown. The deposit structure comprises a DBR with three periods of TiO₂/SiO₂ (168.5/265.4nm), a spacer of SiO₂, a metal layer of Pd, and a protection layer of PTFE with 35nm. Despite using a final polymeric layer, the substrate has a metallic aspect due to the low thickness of PTFE and high thickness of Pd. Figure 10-a-i) is an image of the substrate only with the DBR structure, which presents a red colour when visualized from an angle between 20 and 50°. At normal visualization, the DBR structure appears to be transparent considering that the DBR stopband is in the infrared range. Figure 10-a-ii) shows a Scanning Electron Microscope (SEM) image where the DBR, spacer, Pd, and PTFE layer are perfectly depictable with very defined interfaces and uniformity.

In the TPR structure production, the first step was the DBR deposition. Different substrates were produced simultaneously to guarantee the same structure in this part. The reflectance spectrum of this structure is present in the graph of Figure 10-b) together with the simulated one. Comparing both spectra, it is observed a blueshift on the experimental stopband compared with the numerical. This difference corresponds to an error of 4% on the layer thickness or RI, which considerably impacts the stopband's spectral position. In this way, small experimental errors in the film deposition and/or differences between the RI of the deposit materials and those from the database used for numerical simulations can explain this difference. Using the different substrates with the same DBR structure, three different TPR structures were deposited named Tamm 1, 2, and 3. The Tamm 1 and 2 contain 60 nm of Pd but different spacer thicknesses, namely 250 and 270 nm, respectively. The redshift (approximately 15 nm) of the structure with a thicker spacer confirms the numerical results. The Tamm 3 comprises a structure of 250nm of spacer and 30nm of Pd. In this case, an increase in the minimum reflectivity is observed as expected, increasing from 11% to 24%.

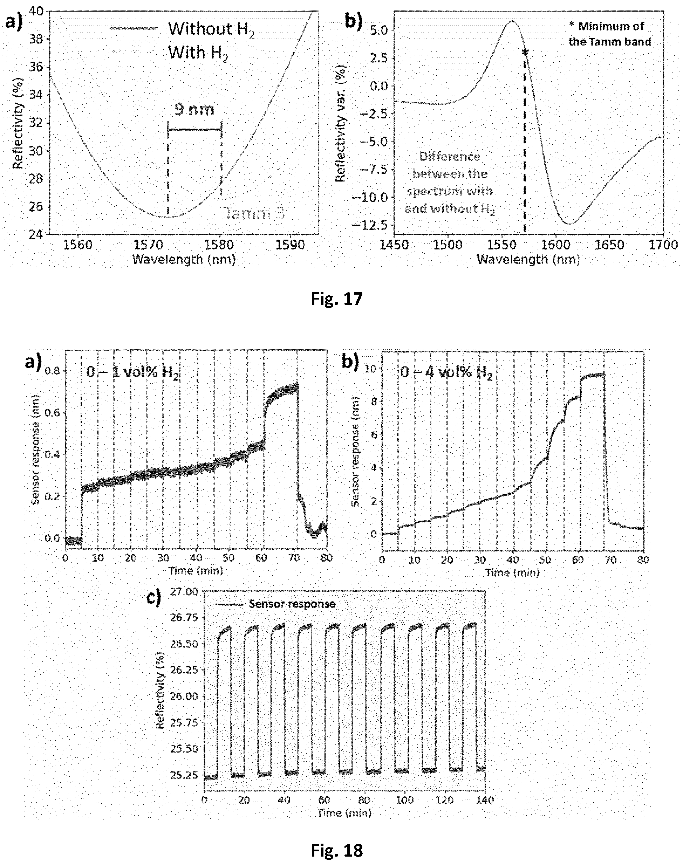

In Figure 10-c), the three TPR bands are present together with the respective bands in the presence of 4 vol% of H₂. In all the bands, it is observed an increase in reflectivity and a redshift of resonance wavelength with a sensitivity of 6, 5 and 9 nm for the Tamm 1, 2, and 3, respectively. Figure 17-a) shows an approximation of the minimum reflectivity for the Tamm 3, where the redshift is easily seen. Figure 17-b) represents the difference between the TPR band with and without H₂. This graph shows reflectivity variations up to 5% or -12.5%, indicating that reflectivity variations can also be used for interrogation and H₂ detection. Despite this, wavelength interrogation will be primarily used due to the better accuracy and to eliminate sources of errors provided by light power fluctuations. Considering the high sensitivity of Tamm 3, this structure is used in the following characterizations.

To better understand the response of the proposed sensor structure to H₂, the TPR band's minimum was analysed over time with a sampling interval of 200ms. The obtained results are represented in **Figure 11****,** where the sensor response corresponds to the wavelength shift of the minimum in relation to the initial wavelength. This figure presents two different cycles, one with 1 and another with 4 vol% H₂. In both cycles, the sensor is initially exposed to the atmospheric air for five minutes, as seen in Figure 11-a), and then the chamber is flushed with 100 vol% N₂. In this transition occurs the purification of the air, with the reduction of the humidity and presence of possible interferents. Despite this, no change in the TPR band was observed. This occurs due to the use of the PTFE layer that protects the Pd layer. Previously published works reported the contraction of Pd and the change in its properties when the air is purified with pure N₂. In this way, this additional layer is important for the sensor's stability. Then, H₂ is introduced in the chamber, where a sensor response of 9.5nm and 0.9nm is obtained for 4 and 1 vol% H₂. At the end, the chamber is again purified with pure N₂, and the TPR band returns to its initial position. The response time, t₉₀, and reset time, t₁₀, were calculated for this sensor as stipulated by the ISO 26142:2010, and the results are presented in Figure 11-b) and c). For the response times, the obtained values were 30s and 25s, while the reset times were 19s and 12s for 4 and 1 vol% H₂, respectively. These values are within the limit of 30s required by the ISO for leaking detection in stationary applications.

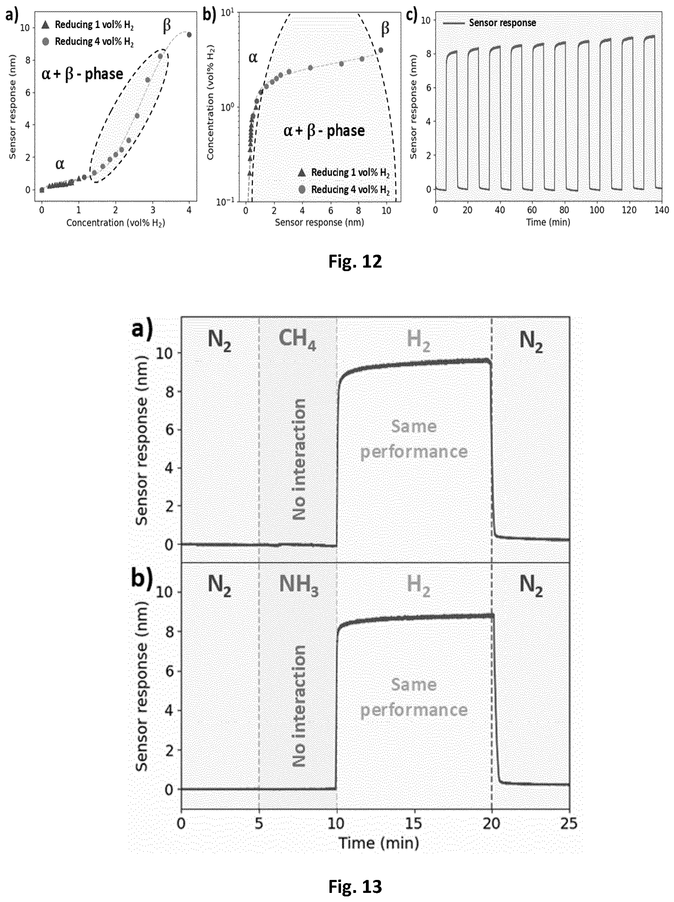

Figure 12-a) represents the sensor response for different H₂ concentrations obtained by diluting 1 and 4 vol% H₂ with pure N₂. The complete timeline with all the data for the different concentrations can be verified in Figure 18-a) and b). With the general overview of the graph from Figure 12-a), the sensor's non-linear response is easily seen. Between 0 and 1 vol%, the sensor response is linear, but between 1 and 3 vol% occurs a transition regime, stabilizing at 4 vol% H₂. This result was expected due to the interaction between the Pd and H₂, and the graph from Figure 12-b) can help with this interpretation.

At low concentrations, the PdHₓ material forms a crystal structure called α-phase. In this phase, the low ratio of H/Pd atoms allows the dilution of the H atoms through the Pd lattice without structure change. However, with the increase of the ratio H/Pd, the interactions H-H start to be dominant, leading to structure changes and the formation of the β-phase. Both phases coexist in this transformation (the nucleation of β-phase), and the materials present a higher mechanical and optical properties variation. When all the α-phase transforms in the β-phase, the change in the optical properties returns to the initial rate. This is one of the disadvantages of using Pd material. Nevertheless, using Pd-alloys with Au, Ag, or Cu can increase the linearity but with a reduction of sensitivity.

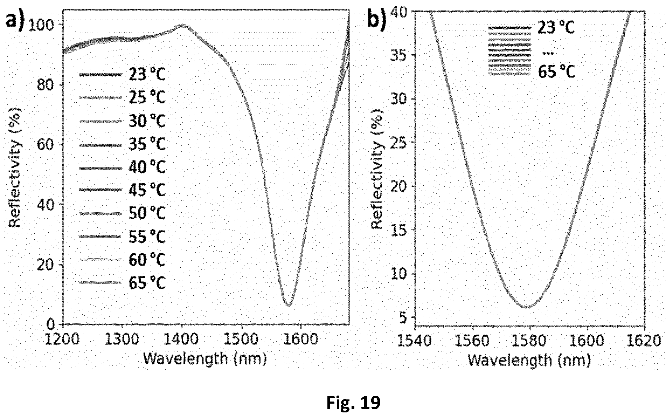

The sensor was exposed to several consecutive cycles with 4 vol% H₂ to evaluate the short-time stability. The results are presented in Figure 12-c), where it is possible to observe the complete reversibility of the sensor response and the stability over time without degradation. Figure 18-c) shows the reflectivity variation for the initial resonance wavelength, with high stability and a reflectivity variation of up to 1.4%. These two graphs confirm the possibility of using two different interrogation techniques, each one with its own advantages. The stability of the sensor when exposed to different temperatures was also tested. The obtained results are presented in Figure 19-a) and b). For temperature variations between 23 and 65°C, the TPR band did not change its resonance wavelength or minimum of reflectivity, staying stable over time. In order to study the impact of interferents, the sensor was exposed to 2500ppm of CH₄ and 500ppm of NH₃, and the results are represented in Figure 13. In both tests, the sensor did not show sensitivity to the interferents, maintaining its initial performance after exposition to these gases.

This disclosure presents an optical H2 sensor based on a plasmonic structure (TPR) with a resonance band at 1572nm and a sensitivity of 9.5nm to 4 vol% of H2. The advantage of the present configuration is the use of cost-effective materials compared with the traditional plasmonic materials (Au and Ag) and the possibility of using normal incidence and depolarized light. In addition, the TPR can be easily tuneable to the desired resonance wavelength over the entire infrared range, as shown in the numerical results present. The sensor presents a response time of up to 30s, a reset time of up to 19s, and high stability in consecutive cycles. The PTFE protection layer ensures protection against the humidity of the atmospheric air, and no cross-sensitivity with CH₄ and NH₃ was observed. With this innovative design and robust performance, this disclosure aims to open new avenues in the field of plasmonic sensors on the infrared regime for remote H₂ detection.

TPR structure deposition: All the materials of this disclosure were deposited using RF-magnetron sputtering with multi-target capability in planar substrates (microscope slides *'Labbox SLIU-005-050'*) and at the tip of optical fibres, from single mode to multimode. The SiO₂ and TiO₂ were deposited with a power of 80W and a rate of 0.04nm/s. The Cr with a power of 30W and a rate of 0.04nm/s, the Pd with a power of 12W and a rate of 0.03nm/s, and the PTFE with a power of 20W and a rate of 0.01nm/s. The deposition of Cr/Pd/PTFE was done in the same vacuum cycle without contact with the atmosphere between depositions to avoid the oxidation of the thin films.

TPR structure characterization: The interrogation of the TPR structure was done using normal and depolarized light. The source used was the *Sarspec* LS-W20 broadband light, and the reflected light was analysed using the spectrometer *OceanOptics* NIRQuest 512. The sensor was characterized using an isolated stainless-steel chamber with a thermal and pressure transducer (KELLER Druck). Inside this external chamber is an internal chamber with a collimator to guarantee the normal incidence of the light. The chambers are connected to a gas system with two inlet lines and one outlet line. The gas flow in the system is controlled by three Thermal Mass Flow controllers (Brooks Instrument model SLA5800). One is used for H₂, another for interferents (CH₄ and NH₃), and another for pure N₂. The gas sources were obtained from the *'Linde'* company, being 1 and 4 vol% of H₂, 0.5vol% CH₄, 10vol% NH₃, and 100vol% N₂. The different concentrations of gas used in this disclosure were obtained by diluting these primary gas sources with N₂. The gas system is controlled by a software that applies and records the data to correlate with the sensor response. The temperature-dependence test was done using a Forced Convention Lab Incubator (Esco Lifesciences) in normal atmospheric conditions.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. Fibre optic sensor for hydrogen plasmonic sensing, comprising an optical fibre having a distal end and a Tamm Plasmon Resonance, TPR, multilayer thin-film stack arranged on the distal end of the optical fibre,
wherein the TPR multilayer thin-film stack comprises:
a periodic arrangement of dielectric material layers forming a Distributed Bragg Reflector, DBR;
a hydrogen-sensitive layer; and
a dielectric spacer layer between the DBR and the hydrogen-sensitive layer;
wherein the plasmonic structure is configured to reflect light according to a resonance condition, and
wherein the hydrogen-sensitive layer is configured to induce a change in said resonance condition according to the concentration of hydrogen gas.

2. The fibre optic sensor according to the previous claim, wherein the Distributed Bragg Reflector comprises two to five periods of dielectric material layers, in particular three periods of dielectric material layers; preferably wherein each period of dielectric material layers comprises a silicon dioxide, SiO₂, layer and a titanium dioxide, TiO₂, layer; more preferably wherein the spacer is a silicon dioxide, SiO₂, layer.

3. The fibre optic sensor according to any of the previous claims, wherein the hydrogen-sensitive layer comprises a metal, or metal alloy, forming a metal hydride in the presence of hydrogen; in particular wherein the metal, or metal alloy, is selected from selective materials such as palladium (Pd), magnesium (Mg), or alloys thereof.

4. The fibre optic sensor according to any of the previous claims, further comprising a protective layer covering the hydrogen-sensitive layer, in particular said protective layer being formed of a polymeric material that is permeable to hydrogen gas.

5. The fibre optic sensor according to any the previous claims, wherein the optical fibre is a single-mode optical fibre.

6. The fibre optic sensor according to any of the previous claims, wherein the measurable change in resonance condition is determined by monitoring variations in the intensity of reflected light at one or more interrogation wavelengths, in particular two interrogation wavelengths; preferably wherein the resonance condition is a resonance band, in particular a frequency-dependent absorption band; more preferably wherein the interrogation wavelengths comprise at least two interrogation wavelengths positioned on opposite slopes of the resonance band.

7. The fibre optic sensor of any one the previous claims, wherein the plasmonic resonance band is located in the infrared spectral region between 1200 nm and 1700 nm.

8. An interrogation system for hydrogen plasmonic sensing, comprising:
at least one fibre optic sensor according to any one of the previous claims;
at least two laser sources, each configured to emit light at a different wavelength, preferably within the range of 1300 nm to 1700 nm;
at least one photodetector configured to receive light reflected from the fibre optic sensor and to generate a corresponding electrical signal.

9. The interrogation system according to the previous claim, further comprising optical switching modules configured to allow selection of one or more of the lasers; and configured to select between a plurality of fibre optic sensors to interrogate.

10. The interrogation system according any of the previous claims, comprising a microcontroller configured to control system operation and communicate with an external computing device; preferably wherein the microcontroller is further configured to manage laser channel selection, optical switching, signal acquisition, and data transmission to the computing device.

11. The interrogation system according to any of the previous claims, further comprising a laser driver circuit configured to supply controlled and stable electrical currents to laser diodes; preferably further comprising a temperature control circuit configured to regulate the temperature in the vicinity of the laser diodes to stabilize their emission wavelengths.

12. An optical sensor for hydrogen plasmonic sensing using infrared wavelengths, comprising a Tamm Plasmon Resonance, TPR, multilayer thin-film stack;
wherein the TPR multilayer thin-film stack comprises:
a periodic arrangement of dielectric material layers forming a Distributed Bragg Reflector, DBR;
a hydrogen-sensitive layer; and
a dielectric spacer layer between the DBR and the hydrogen-sensitive layer;
wherein the plasmonic structure is configured to reflect light according to a resonance condition, and
wherein the hydrogen-sensitive layer is configured to induce a change in said resonance condition according to the concentration of hydrogen gas;
wherein the DBR comprises two to five periods of dielectric material layers, preferably three periods of dielectric material layers;
wherein each period of dielectric material layers comprises a silicon dioxide, SiO₂, layer and a titanium dioxide, TiO₂, layer.

13. The optical sensor according to the previous claim, wherein the spacer is a silicon dioxide, SiO₂, layer.

14. The optical sensor according to any of the claims previous claims 12 or 13, wherein the hydrogen-sensitive layer comprises a metal, or metal alloy, forming a metal hydride in the presence of hydrogen; in particular wherein the metal, or metal alloy, is selected from palladium (Pd), magnesium (Mg), or alloys thereof.

15. The optical sensor according to any of the claims 12-14, further comprising a protective layer covering the hydrogen-sensitive layer, in particular said protective layer being formed of a polymeric material that is permeable to hydrogen gas.
